# EUROPEAN PATENT SPECIFICATION

(11) **EP 4 437 082 B1**
(45) Date of publication and mention of the grant of the patent: **27.08.2025**
(21) Application number: 22835911.3
(22) Date of filing: 08.12.2022
(51) Int. Cl.: C12N 1/20, A01N 63/25

(54) **A NOVEL PAENIBACILLUS POLYMYXA STRAIN AND THE USE OF THE NOVEL PAENIBACILLUS POLYMYXA STRAIN**
NEUER PAENIBACILLUS-POLYMYXA-STAMM UND VERWENDUNG DES NEUARTIGEN PAENIBACILLUS-POLYMYXA-STAMMS
NOUVELLE SOUCHE DE PAENIBACILLUS POLYMYXA ET UTILISATION DE LA NOUVELLE SOUCHE DE PAENIBACILLUS POLYMYXA

(30) Priority: 22.12.2021 PL 43994821
(43) Date of publication of application: 02.10.2024
(73) Proprietor: INTERMAG SP. Z O.O, 32-300 Olkusz (PL)
(72) Inventor: KARDASZ, Hubert, 32-300 Olkusz (PL); HALAT-LAS, Malgorzata, 32-010 Luczyce (PL); OLESZCZAK, Marcin, 25-640 Kielce (PL); KAFEL, Ilona, 32-045 Soluszowa (PL); JOPEK, Magdalena, 32-020 Wieliczka (PL); AMBROZIAK, Krzysztof, 31-345 Kraków (PL); RAKOCZY, Roksana, 32-070 Czernichów (PL)
(74) Representative: JWP Patent & Trademark Attorneys
(86) International application number: PCT/PL2022/050090
(87) International publication number: WO 2023/121491

(56) References cited:
- LIU XIAOMENG ET AL: "Paenibacillus strains with nitrogen fixation and multiple beneficial properties for promoting plant growth", PEERJ, vol. 7, 1 January 2019 (2019-01-01), pages 1 - 19, XP055964773, Retrieved from the Internet <URL:https://peerj.com/articles/7445.html> DOI: 10.7717/peerj.7445
- HUSSAIN AZHAR ET AL: "Plant-growth-promoting Bacillus and Paenibacillus species improve the nutritional status of Triticum aestivum L.", PLOS ONE, vol. 15, no. 12, 1 December 2020 (2020-12-01), pages e0241130, XP093019546, Retrieved from the Internet <URL:https://journals.plos.org/plosone/article/file?id=10.1371/journal.pone.0241130&type=printable> DOI: 10.1371/journal.pone.0241130
- ELLIOT NICHOLAS GRADY ET AL: "Current knowledge and perspectives of Paenibacillus: a review", MICROBIAL CELL FACTORIES, vol. 15, no. 1, 1 December 2016 (2016-12-01), XP055376371, DOI: 10.1186/s12934-016-0603-7

## Description

### Technical Field

The subject of the present invention is a novel *Paenibacillus polymyxa* strain and the use of the novel *Paenibacillus polymyxa* strain in plant cultivation.

### Background Art

Due to increasing restrictions on the use of chemical plant protection products, including a decreasing number of active ingredients authorized for use in agriculture, the sector of biological preparations containing novel bacterial strains is increasingly growing in importance.

Nitrogen is one of the key elements affecting the growth and development of plants, including also cultivated plants. In plants, nitrogen deficiency causes stunted growth, leaf yellowing, lack of vigour, lower yields and, in many cases, worse yield quality, and premature fruit ripening. Nitrogen fertilizers are routinely used in agricultural practice in order to provide optimum plant growth and attain high productivity. However, more than a half of the nitrogen supplied from the synthetic nitrogen fertilizers used is not utilized by the cultivated plants, and instead is lost to the environment, where it contributes to the production of greenhouse gases, acid rain and biodiversity loss in marine ecosystems. In accordance with the Regulation of the Council of Ministers of 12 February 2020 on the adoption of a "Programme of measures to reduce water pollution by nitrates from agricultural sources and to prevent further pollution" (Journal of Laws 2020, item 243), artificial and natural nitrogen fertilizers can be applied to fields only within strictly defined time frames. Therefore, biological forms of plant fertilization that rely on the potential of microorganisms, which includes, among others, the ability to bind atmospheric nitrogen, are becoming increasingly popular.

Phosphorus, like nitrogen, is a very important macronutrient essential for growth, improved health and high yields of plants. Phosphorus may be present in the soil in the form of organic and mineral compounds. Individual phosphorus compounds are characterized by highly varied solubility in water and in weak acids, thus their availability to plants varies as well. The availability of this mineral to plants is determined by the soil content of phosphorus compounds soluble in weak acids. Phosphorus available to plants constitutes only 2 to 10% of the total phosphorus contained in the soil. In agricultural practice, phosphate-based fertilizers are routinely applied to the soil. However, the phosphorus fertilizer applied can be quickly immobilized as a result of the precipitation reaction with aluminium and calcium contained in the soil. Consequently, it becomes unavailable to plants, thereby constituting a soil reserve of this nutrient that is of greatly limited availability to plants. Soil erosion also leads to the loss of phosphorus from the soil, whereupon it typically accumulates in lakes and rivers, causing water eutrophication. The utilization of microbes having the ability to solubilize phosphorus, i.e. increase its solubility, and thus its soil availability to plants, appears to be an ideal alternative to mineral phosphorus fertilizers.

Zinc is an element that significantly improves plant resistance to diseases, while also increasing the effectiveness of nitrogen fertilization. It is involved in numerous physiological processes occurring within the plant. Its numerous properties have a beneficial effect on the amount of yield obtained. In order to be able to provide an adequate level of plant nutrition with this element, foliar fertilization is applied. Zinc is a micronutrient that is essential for the proper growth and development of plants. It has a very good effect on improving resistance to diseases, among others: the resistance of potatoes to scab and the resistance of wheat to stem base blight. Its properties also help during cultivation and fertilization of maize. Adequate nutrition with zinc in the early stages of maize development, combined with an increase in plant hormone activity, has an effect on improving the effectiveness of nitrogen fertilization, and in later stages it increases the efficiency of carbon dioxide binding. Thanks to its numerous properties, zinc has a beneficial effect on the amount of yield, therefore good plant nutrition with this micronutrient should be ensured from the very beginning of vegetation. Zinc fertilization is often overlooked in practice, with zinc deficiencies resulting in extensive pathological lesions in plants. A solution to this problem appears to be the use of microorganisms that enable plants to access zinc from forms found in the soil that are unavailable to plants.

In agricultural practice, silicon-based fertilizers are also increasingly used. Silicon is one of the most common elements on Earth. It accounts for about twenty percent or so of the lithosphere and is a constituent of more than 300 minerals found in nature. It is one of the basic soil components, as it is a component of nearly all the parent rocks from which soils are formed. In temperate climate soils, silicon is typically present in the solid phase of the soil in the form of primary and secondary minerals (silicates and quartz), with a small amount of silicon being present in the soil in the form of plant residues or microorganisms. The total silicon content in soils is very high, usually amounting to about 30%. The primary source of silicon available to plants in soils are the weathering processes of primary and secondary silicates and aluminosilicates, where rocks composed of minerals rich in silicon compounds undergo weathering under the influence of environmental factors, which leads to the formation of silicon compounds with different types of crystallization, and free silica. Water-soluble silicon compounds play a huge role in the process of enabling plants to access certain elements from the soil. The utilization of microbes having the ability to solubilize Si, that is to increase its solubility, and thus its soil availability to plants, appears to be an ideal way of replenishing the Si content of soils and plants.

Microbiological preparations containing, among others, symbiotic leguminous bacteria obtained on the basis of bacteria of the Rhizobium group, or vaccines mycorrhizing tree seedlings, produced on the basis of the Trichoderma spp. fungi, are known and used in agriculture. Products containing microorganisms such as *Alternaria, Trichoderma, Azotobacter, Bacillus, Pseudomonas, Streptomyces* and others are also used for biological plant protection.

A biopreparation for increasing plant yields and protecting plants against diseases is known from the patent description RU2147181 C1 which comprises a hydrolyzate of the *Pseudomonas aureofaciens* VKM V-1973D bacteria in an amount of 18 - 20 parts by weight, a hydrolyzate of the *Bacillus megaterium* bacteria in an amount of 39 - 40 parts by weight, a pine needle extract in an amount of 5 - 6 parts by weight, a chlorophyll and carotene paste in an amount of 1 - 2 parts by weight, and a macro- and micronutrient solution in an amount of 32 - 37 parts by weight.

A biopreparation intended for controlling fungi of the *Fusarium* genus that cause plant diseases is known from the patent description RO126081 B1 which consists of a 1 - 20% suspension of microorganisms: *Saccharomyces cerevisiae* L30 (NCAIM deposit number Y001350) and *Bacillus subtilis* B49b (NCAIM deposit number (P) 001360 B), preferably between 4 - 8% of each strain, 2 - 15% of nutrients, 5 - 15% of surfactants, 10 - 50% of the solvent, 0,01 - 1% of a stabilizer, 1 - 10% of anticoagulants, and distilled water in an amount of up to 100%.

In recent years, increasingly numerous reports have appeared on the use of bacteria of the *Paenibacillus polymyxa* genus in plant cultivation. In a publication by B. Weselowski et al., entitled: "Isolation, identification and characterization of Paenibacillus polymyxa CR1 with potentials for biopesticide, biofertilization, biomass degradation and biofuel production", BMC Microbiol, 2016 Oct 18;16(1):244, they are described as plant growth-promoting bacteria distinguished by their versatile activity. They have the ability to solubilize phosphorus, produce plant hormones or degrade lignocellulose. Their unique feature is also their potential ability to bind atmospheric nitrogen, which makes them an extremely interesting object of study, because it is very rare for sporulating bacteria to exhibit the ability to synthesize the enzyme responsible for the process of atmospheric nitrogen reduction. The aforementioned publication also describes the antagonistic activity of the *Paenibacillus polymyxa* CR1 strain against fungi: *Phytopthora sojae* and *Rhizoctonia solan.*

Similar results have been obtained for the *Paenibacillus polymyxa* ShX301 strain capable of inhibiting the mycelium development in the following species: *Phytophthora capsici, Fusarium oxysporum, Colletotrichum truncatum, Botrytis cinerea, Sclerotinia sclerotiorum* and *Rhizoctonia solani,* as described in a publication by F. Zhang et al., entitled: "Biocontrol potential of Paenibacillus polymyxa against Verticillium dahliae infecting cotton plants".

Strains: *Paenibacillus polymyxa* Sx3 and *Paenibacillus polymyxa* ATCC 842, capable of solubilizing phosphorus and binding nitrogen, are known from a publication by Y. Abdallah et al., entitled: "Plant growth promotion and suppression of bacterial leaf blight in rice by Paenibacillus polymyxa Sx3" Letters in Applied Microbiology 68, 423-429, and by E. A. Finch et al., entitled: "Effects of Paenibacillus polymyxa inoculation on below-ground nematode communities and plant grow", Soil Biology and Biochemistry, 121 (2018) 1-7.

A *Paenibacillus polymyxa* QZY-1 strain, capable of solubilizing phos-phorus and having antagonistic properties against plant pathogens: *Ralstonia solanacearum and Burkholderia cepacia,* is known from the patent application no. CN110684695 A.

Strains of the *Paenibacillus polymyxa* bacteria that have a proven antagonistic activity against bacterial and fungal plant pathogens are also known from the patent applications CN111548976 A and CN112795507 A.

There are a number of preparations on the market that rely on the potential of microorganisms, however, many of these solutions exhibit drawbacks such as insufficient durability, low effectiveness and a narrow range of activity.

The aim of the present invention is to isolate a novel strain of bacteria of the Paenibacillus polymyxa species, with a broad activity and long durability, the utilization of which in plant cultivation may be a solution to the drawbacks indicated above.

### Summary of Invention

The essence of the present invention is a novel *Paenibacillus polymyxa* B134 strain, PCM deposit no. B/00335, deposited in the Polish Collection of Microorganisms at the Institute of Immunology and Experimental Therapy of the Polish Academy of Sciences in Wroclaw.

The essence of the present invention is also the use of the novel *Paenibacillus polymyxa* B134 strain, PCM deposit no. B/00335, deposited in the Polish Collection of Microorganisms of the Polish Academy of Sciences in Wroclaw, for use against fungal pathogens and for biostimulation of plant growth and development.

The *Paenibacillus polymyxa* B134 strain, which is the subject of the present invention, is distinguished by its versatile activity. In addition to the biostimulating properties, i.e. the solubilization of silicon, zinc and phosphorus, and the binding of nitrogen, this strain is characterized by antagonistic activity against plant pathogens, especially fungal pathogens. The conducted tests showed that the novel *Paenibacillus polymyxa* B134 strain exhibited an exceptionally potent antagonistic activity and effectiveness in suppressing the mycelium development against as many as 14 tested fungal plant pathogens. Combining the above-mentioned features with high durability and stability of the spore-forming *Paenibacillus polymyxa* B134 bacteria will yield a preparation that, in addition to the broad spec-trum of activity, will be characterized by resistance to transport conditions and a long period of storage stability.

### Identification of the strain

The *Paenibacillus polymyxa* B134 strain was deposited in the Polish Collection of Microorganisms (PCM) at the Institute of Immunology and Experimental Therapy of the Polish Academy of Sciences, 53-114 Wroclaw, ul. Rudolfa Weigla 12, in accordance with the Budapest Treaty. The deposit was made on 18.05.2021. The deposit was numbered as follows: *Paenibacillus polymyxa* B134, deposit number B/00335.

### Method of isolating the Paenibacillus polymyxa B134 strain

The *Paenibacillus polymyxa* B134 strain was isolated from soil obtained from the environment. Soil samples with a weight of 10 g were suspended in 90 ml of sterile distilled water and then vigorously stirred. The sample was heated at 70°C for 15 minutes. A series of dilutions of the tested sample (up to 10-9) was prepared from the obtained suspension. From each dilution, 0.1 ml was taken and surface inoculation was performed on a solidified 1/5 NPT medium. The composition of the culture medium is shown in Table 1. All components of the culture media were dissolved in H₂O, their pH was adjusted to 5.75 and they were autoclaved at 121°C for 15 min.

The plates were incubated at 28°C for 72h. Single colonies were streaked onto a suitable solid medium. The plates were incubated at 28°C for 72h. In order to obtain pure colonies, the procedure was repeated several times.

The isolated pure *B. polymyxa* colonies were transferred into tubes containing a liquid NPT culture medium. They were incubated for 24h at 28°C. The cells were stored using ready-made cryovials (Microbank, Biocorp) intended for storing bacterial and fungal cultures.

**[Table 1]**

| Component | Liquid medium | Solid medium |
|---|---|---|
| Nutrient broth | 0.4 g | 0.4 g |
| Glucose | 1 g | 1 g |
| Trypsin soy hydrolyzate | 1.2 g | 1.2 g |
| MES hydrate | 2 g | 2 g |
| Agar | - | 15 g |
| Water | 1 dm3 | 1 dm3 |

Determination of the species affiliation of the *Paenibacillus polymyxa* B134 strain.

The species affiliation of the *Paenibacillus polymyxa* B134 strain was determined by genotypic methods (the PCR method). Sequencing of the 16S rRNA subunit was performed and then the obtained sequence was then compared with the NCBI ( *National Center for Biotechnology Information*) database. As a result of the conducted analysis, it was demonstrated that the strain belongs to the following species: *Paenibacillus polymyxa.*

### Description of Embodiments

The present invention is shown in the following embodiments and its effectiveness is demonstrated in exemplary wheat and maize cultures.

### Example 1

Determination of the ability of the *Paenibacillus polymyxa* B134 strain to bind nitrogen.

The presence of the nitrogenase-coding gene, i.e. the enzyme responsible for binding nitrogen, was determined genetically by sequencing and analyzing a fragment of the *nif*H sequence.

The ability of the *Paenibacillus polymyxa* B134 strain to bind nitrogen was determined qualitatively by the scale method, through incubation on a medium containing no nitrogen source. The microorganism growth under nitrogen-free conditions was indicative of the ability to bind atmospheric nitrogen.

Determination of the ability of the *Paenibacillus polymyxa* B134 strain to solubilize phosphorus, zinc and silicon.

In order to determine the ability of the *Paenibacillus polymyxa* B134 strain to solubilize phosphorus, zinc and silicon, experiments were performed using specific culture media with the compositions given in Tables 2, 3, 4.

Table 2 shows the composition of the medium for testing the ability to sol-ubilize phosphorus, Table 3 - the composition of the medium for testing the ability to solubilize zinc, and Table 4 - the composition of the medium for testing the ability to solubilize silicon.

Bacteria were grown on a solid TSA medium at 30°C for 48h. The bacterial colonies that grew on the solid medium were used to inoculate a liquid TSB culture medium contained in a tube. In the next step, the contents of the tube were employed to inoculate a liquid TSB culture medium contained in a 250 ml conical flask. The culture was carried out on a rotary shaker at 30°C. Samples for activity testing were taken after 48h. The tests employed a cellular biomass that was prepared as follows: The cultures were centrifuged, the supernatant was removed and the precipitate was suspended in the same volume of sterile saline. 10 µl of bacterial suspension was applied on the surface of each of the solidified media shown in Tables 2, 3, 4. The plates were incubated in a thermostated incubator at 30°C.

The diameter of the halo and the resulting colony were measured after 2 and 7 days for the tests concerning the solubilization of zinc, after 4 and 7 days for silicon, and after 14 days for the solubilization of phosphorus. The tests were performed in two independent biological replicates.

**[Table 2]**

| Component | Amount [g] |
|---|---|
| Glucose | 10 |
| Ca3(PO4)2 | 5 |
| MgSO4 ·7H2O | 0.1 |
| NaCl | 0.2 |
| (NH4)2SO4 | 0.5 |
| KCl | 0.2 |
| MnSO4 ·H2O | 0.002 |
| FeSO4 ·7H2O | 0.002 |
| Agar | 15 |
| Distilled water | 1 dm3 |

**[Table 3]**

| Component | Amount [g] |
|---|---|
| Glucose | 10 |
| (NH4)2SO4 | 1 |
| KCl | 0.2 |
| K2HPO4 | 0.1 |
| MgSO4 | 0.2 |
| ZnO | 1 |
| Agar | 15 |
| Distilled water | 1 dm3 |

**[Table 4]**

| Component | Amount [g] |
|---|---|
| Glucose | 10 |
| (NH4)2SO4 | 1 |
| KCl | 0.2 |
| K2HPO4 | 0.1 |
| MgSO4 | 0.2 |
| Mg2O8Si3 | 2.5 |
| Agar | 15 |
| Distilled water | 1 dm3 |

The *Paenibacillus polymyxa* B134 strain produced a halo zone around the colony, thus demonstrating the ability to solubilize phosphorus, silicon and zinc.

### Example 2

Determination of the antagonistic properties of the *Paenibacillus polymyxa* B 134 strain against selected fungal pathogens.

Determination of the antagonism against fungal pathogens was conducted in laboratory tests. A disc with a mycelium, cut using a corkborer, was applied onto PDA (Potato-Dextrose Agar) agar plates at a distance of 2 cm from the edge. Fungal isolates were incubated at standard temperature for a period of 1-5 days until the mycelium reached the center of the plate. Then, a fresh bacterial suspension with a volume of 10 µL was distributed at a distance of 5 cm from the disc with the mycelium. The plates were then incubated at room temperature for 14 days, with a reading performed after 7 and 14 days. The antagonistic activity was determined based on the occurrence (or lack thereof) of a mycelium growth inhibition zone in area of bacterial growth. For the tests, 14 pathogen isolates were employed:
1. **IOR collection number:** 2216 **Pathogen species:** *Fusarium oxysporum*
2. **IOR collection number:** 2204 **Pathogen species:** *Rhizoctonia solani*
3. **IOR collection number:** 2172 **Pathogen species:** *Fusarium graminearum*
4. **IOR collection number:** 2170 **Pathogen species:** *Fusarium avenaceum*
5. **IOR collection number:** 2105 **Pathogen species:** *Fusarium culmorum*
6. **IOR collection number:** 1756 **Pathogen species:** *Cladosporium cladosporioides*
7. **IOR collection number:** 1665 **Pathogen species:** *Fusarium poae*
8. **IOR collection number:** 1112 **Pathogen species:** *Septoria nodorum*
9. **IOR collection number:** 582 **Pathogen species:** *Septoria tritici*
10. **IOR collection number:** 412 **Pathogen species:** *Pseudocercosporella* herpotrichoides
11. **IOR collection number:** 934 **Pathogen species:** *Microdochium nivale*
12. **IOR collection number:** 2284 **Pathogen species:** *Phoma lingam*
13. **IOR collection number:** 2233 **Pathogen species:** *Alternaria brassicae*
14. **IOR collection number:** 2182 **Pathogen species:** *Sclerotinia sclerotiorum*

As a result of the conducted experiments, the antagonistic activity of the *Paenibacillus polymyxa* B134 strain against each of the aforementioned 14 tested fungal pathogens was demonstrated. In each case, the inhibition of mycelium growth occurred a dozen or even several dozens of mm before the site of bacterial growth, which is indicative of the exceptionally potent antagonistic activity of the novel *Paenibacillus polymyxa* B134 strain.

### Example 3

Determination of the *in situ* biostimulating properties of the *Paenibacillus polymyxa* B134 strain.

The *Paenibacillus polymyxa* B134 strain was used in the cultivation of wheat and maize, and tests concerning its effect on the basic, important parameters of these plants were conducted.

At the beginning of the experiment, a peat substrate was inoculated with the tested *Paenibacillus polymyxa* B134 microorganisms and with Standard I and Standard II reference preparations, and with water (control), then seeds were planted and the following were assessed:
a. the number of emergences (germination dynamics),
b. plant height,
c. plant weight.

The experiment was carried out in a foil tunnel. Table 5 shows the wheat and maize emergence score with respect to control, Table 6 - the wheat height and weight with respect to control, and Table 7 - the maize height and weight with respect to control.

**[Table 5]**

| | Wheat | | Maize | |
|---|---|---|---|---|
| Microorganisms | Emergences with respect to control [%] Day 5 | Emergences with respect to control [%] Day 6 | Emergences with respect to control [%] Day 5 | Emergences with respect to control [%] Day 6 |
| Paenibacillus polymyxa B134 | 367 | 136 | 143 | 123.33 |
| Standard I | 312 | 131 | 138 | 118 |
| Standard II | 312 | 131 | 172 | 150 |
| Control | 100 | 100 | 100 | 100 |

**[Table 6]**

| Wheat | | | | |
|---|---|---|---|---|
| Microorganisms | Plant height with respect to control [%] Day 7 | Plant height with respect to control [%] Day 9 | Plant height with respect to control [%] Day 12 | Weight with respect to control [%] |
| Paenibacillus polymyxa B134 | 130 | 124 | 109 | 108 |
| Standard I | 124 | 121 | 103 | 100 |
| Standard II | 130 | 118 | 104 | 92 |
| Control | 100 | 100 | 100 | 100 |

**[Table 7]**

| Maize | | | | |
|---|---|---|---|---|
| Microorganisms | Plant height with respect to control [%] Day 7 | Plant height with respect to control [%] Day 9 | Plant height with respect to control [%] Day 12 | Weight with respect to control [%] |
| Paenibacillus polymyxa B134 | 112 | 129 | 113 | 110 |
| Standard I | 106 | 113 | 115 | 114 |
| Standard II | 112 | 116 | 111 | 107 |
| Control | 100 | 100 | 100 | 100 |

The results of the conducted experiments clearly indicate the positive effect of the *Paenibacillus polymyxa* B134 strain on the tested plant parameters, both in the case of wheat and maize.

The conducted tests demonstrated that the novel *Paenibacillus polymyxa* B134 strain can be regarded as a strain with biostimulating properties for the growth and development of cultivated plants. Its properties were confirmed in laboratory tests, while the effect on plants was demonstrated in tunnel tests.

## Claims

1. A novel Paenibacillus polymyxa B134 strain, PCM deposit no. B/ 00335, deposited in the Polish Collection of Microorganisms of the Polish Academy of Sciences in Wroclaw.

2. The use of the novel Paenibacillus polymyxa B134 strain, PCM deposit no. B/00335, deposited in the Polish Collection of Microorganisms of the Polish Academy of Sciences in Wroclaw, for use against plant pathogens and for fertilization and biostimulation of plant growth and development.

## Patentansprüche

1. Ein neuer Paenibacillus polymyxa B134-Stamm, PCM-Hinterlegungsnr. B/00335, hinterlegt bei der Polnischen Sammlung von Mikroorganismen der Polnischen Akademie der Wissenschaften in Breslau.

2. Die Verwendung des neuen Paenibacillus polymyxa B134-Stammes, PCM-Hinterlegungsnr. B/00335, hinterlegt bei der Polnischen Sammlung von Mikroorganismen der Polnischen Akademie der Wissenschaften in Breslau, zur Verwendung gegen Pflanzenpathogene und zur Befruchtung und Biostimulation von Pflanzenwachstum und -entwicklung.

## Revendications

1. Nouvelle souche bactérienne de Paenibacillus polymyxa B134, dépôt PCM no. B/00335, déposée à la Collection polonaise de Microorganismes de l'Académie Polonaise des Sciences à Wroctaw.

2. Utilisation de nouvelle souche bactérienne de Paenibacillus polymyxa B134, dépôt PCM no. B/00335, à la Collection polonaise de Microorganismes de l'Académie Polonaise des Sciences à Wroctaw, pour utiliser contre des agents phytopathogènes et pour la fertilisation et la phytostimulation de croissance et du développement des plantes.
